# EUROPÄISCHE PATENTSCHRIFT

(11) **EP 2 654 600 B1**
(45) Veröffentlichungstag und Bekanntmachung des Hinweises auf die Patenterteilung: **21.02.2018**
(21) Anmeldenummer: 11799279.2
(22) Anmeldetag: 13.12.2011
(51) Int. Cl.: A61C 7/36, A61C 7/08

(54) **KIEFERORTHOPÄDISCHER APPARAT**
ORTHODONTIC APPARATUS
APPAREIL ORTHOPÉDIQUE DE MAXILLAIRE

(30) Priorität: 23.12.2010 DE 202010017014 U
(43) Veröffentlichungstag der Anmeldung: 30.10.2013
(73) Patentinhaber: Köklü, Saduman Oguzhan, 58332 Schwelm (DE)
(72) Erfinder: Köklü, Saduman Oguzhan, 58332 Schwelm (DE)
(74) Vertreter: dompatent von Kreisler Selting Werner - Partnerschaft von Patent- und Rechtsanwälten mbB
(86) Internationale Anmeldenummer: PCT/EP2011/072644
(87) Internationale Veröffentlichungsnummer: WO 2012/084611

(56) Entgegenhaltungen:
- WO-A2-02/26155
- DE-U1-202008 010 330
- DE-U1-202008 016 419
- US-A- 5 829 441
- US-A1- 2003 217 753
- US-A1- 2007 235 037

## Beschreibung

Die Erfindung betrifft einen kieferorthopädischen Apparat mit einer Oberkieferschiene und einer Unterkieferschiene, wobei mindestens eine dieser Kieferschienen ein Angreifteil aufweist, welches an der Kieferschiene mit einem Verstellmechanismus in sagittaler Richtung verstellbar ist.

Ein kieferorthopädischer Apparat, von dem der Oberbegriff des Anspruchs 1 ausgeht, ist beschrieben in DE 20 2008 016 419 U (Köklü). Dieser Apparat weist eine Oberkieferschiene und eine Unterkieferschiene auf. Beide Kieferschienen haben Angreifteile, die beim Schließen des Mundes zusammenwirken, um die Stellung des Unterkiefers in Bezug auf den Oberkiefer zu korrigieren. Dabei kann jedes der beiden Angreifteile an der zugehörigen Kieferschiene verstellbar sein.

Es reicht jedoch aus, wenn eines der Angreifteile verstellbar ist, während das andere an seiner Kieferschiene fest ist. Der Verstellmechanismus des einen Angreifteiles hat bei dem bekannten kieferorthopädischen Apparat eine Schraubvorrichtung in Form einer Hyrax®-Schraube. Eine solche Verstellvorrichtung befindet sich vollständig zwischen den beiden Schenkeln der betreffenden Kieferschiene. Zum Verstellen des Angreifteiles muss die Kieferschiene aus dem Mund herausgenommen werden. Dann muss die mit gegenläufigen Gewindeabschnitten versehene Spindel durch seitliches Ansetzen eines stiftförmigen Werkzeuges gedreht werden, wobei das Werkzeug in Querbohrungen der Spindel eingesetzt wird. Eine solche Struktur erlaubt nur das abschnittsweise Drehen der Spindel jeweils nur um einen begrenzten Drehwinkel. Das Verstellen des Angreifteiles an der Kieferschiene ist daher mühsam und langwierig. Außerdem ist eine Verstellung nicht im Munde des Patienten möglich, so dass der Kieferorthopäde die Passgenauigkeit der jeweiligen Einstellung nicht in situ überprüfen kann.

US 2003/0217753 A1 und US 2007/0235037 A1 beschreiben jeweils kieferorthopädische Apparate mit einem Verstellmechanismus, der eine Kopplungsstruktur zum Ansetzen eines Drehwerkzeuges aufweist.
Der Erfindung liegt die Aufgabe zugrunde, einen kieferorthopädischen Apparat zu schaffen, bei dem die Verstellung bzw. Einstellung des Angreifteiles vereinfacht ist, und das eine bessere und schnellere Kontrolle durch den Kieferorthopäden ermöglicht.
Der kieferorthopädische Apparat der vorliegenden Erfindung ist durch den Anspruch 1 definiert. Die Stellspindel des Verstellmechanismus weist ein Schaftende auf, das am frontalen Bogenbereich der Kieferschiene freiliegt und dort eine zum frontalen Ansetzen eines Drehwerkzeugs geeignete Kopplungsstruktur aufweist.

Der erfindungsgemäße kieferorthopädische Apparat erleichtert wesentlich das Einstellen und die Kontrolle der zusammenwirkenden Angreifteile von Oberkieferschiene und Unterkieferschiene. Insbesondere braucht zum Durchführen der Verstellung keine der beiden Kieferschienen aus dem Mund des Patienten herausgenommen zu werden, weil der Verstellmechanismus von vorne mit dem Drehwerkzeug zugänglich ist, und zwar selbst dann, wenn die Angreifteile beider Kieferschienen in gegenseitigem Eingriff sind. Hierdurch wird eine in situ-Einstellung des kieferorthopädischen Apparates ermöglicht, wobei der Patient während des Verstellvorganges gefragt werden kann, ob er Druck oder andere Unannehmlichkeiten spürt. Erfindungsgemäß ist die Kopplungsstruktur in dem Material der Kieferschiene versenkt angeordnet, ohne über die Kontur dieser Kieferschiene hinaus vorzustehen. Damit ist sichergestellt, dass der Komfort des Trägers nicht durch überstehende Teile beeinträchtigt wird. Außerdem ist die Kopplungsstruktur geschützt in der jeweiligen Kieferschiene angeordnet.
Die Kopplungsstruktur ist in der Regel ein Innenmehrkant, beispielsweise ein Innensechskant oder Innenvierkant. Dies hat den Vorteil, dass ein Spindelkopf mit zylindrischer Außenfläche verwendbar ist, bei dem ein Spalt zwischen dem Spindelkopf und dem Kunststoffmaterial der Kieferschiene auf null reduziert ist.
Im Folgenden wird unter Bezugnahme auf die Zeichnungen ein Ausführungsbeispiel der Erfindung näher erläutert.

Es zeigen:
- Figur 1: eine perspektivische Ansicht einer Oberkieferschiene,
- Figur 2: eine perspektivische Ansicht einer Unterkieferschiene,
- Figur 3: eine Draufsicht der Unterkieferschiene aus Richtung des Pfeiles III von Figur 2,
- Figur 4: eine Draufsicht der Oberkieferschiene aus Richtung des Pfeiles IV in Figur 1,
- Figur 5: eine perspektivische Ansicht eines weiteren Ausführungsbeispiels einer Unterkieferschiene, und
- Figur 6: eine perspektivische Ansicht eines weiteren Ausführungsbeispiels einer Unterkieferschiene.

Der kieferorthopädische Apparat weist eine Oberkieferschiene OKS und eine Unterkieferschiene UKS auf. Beide Schienen sind nach Art der Zahnspangen ausgebildet und haben daher eine im Wesentlichen U-förmige Grundform. Die Schienen werden der Kieferform und Zahnform des Patienten im Abdruckverfahren angepasst, so dass sie störungsfrei und herausnehmbar im Mund eines Patienten getragen werden können. Die Oberkieferschiene OKS weist einen frontalen Bogenbereich 10 sowie zwei Seitenschenkel 11 und 12 auf. Von der Rückseite des Bogenbereichs 10 steht der Verstellmechanismus 13 ab, der ein hakenförmiges Angreifteil 14 trägt, dessen Mittelachse in der Medianebene der Kieferschiene liegt. Das Angreifteil 14 ist Bestandteil eines aus Kunstharz geformten Kopfstücks 15, dessen Position durch Betätigung des Einstellmechanismus 13 nach vorne (frontal) bzw. hinten (dorsal) stufenlos verändert werden kann. Im Kopfstück 15 befindet sich eine (nicht dargestellte) Spindelmutter, die eine schraubenförmige Spindel 16 aufnimmt, welche in der Medianebene der Kieferschiene verläuft. Am vorderen Ende der Spindel 16 befindet sich eine Kopplungsstruktur 17, an der ein Werkzeug T angesetzt werden kann, um die Spindel 16 zu drehen. Durch Drehen der Spindel wird das Angreifteil 14 vor- bzw. zurückbewegt.

Die Spindelmutter lagert zwei parallel abstehende Führungsstifte 18, deren vordere Enden durch ein Joch 19 verbunden sind. In dem Joch 19 ist der die Kopplungsstruktur bildende Spindelkopf 17 gelagert. Das Joch ist ferner mit zwei aus biegsamem Draht bestehenden Armen 20 verbunden, welche in das Kunstharzmaterial der Kieferschiene eingebettet sind. Die Arme bewirken eine Verankerung und Drehsicherung des Verstellmechanismus 13 in Bezug auf die Kieferschiene. Die Führungsstifte 18 gehen in zwei aus biegsamem Draht bestehende Arme 21 über, welche in das Kunstharzmaterial des hakenförmigen Angreifteils 14 eingebettet sind.

Der Verstellmechanismus 13 ist ein Produkt der Firma Forestadent.

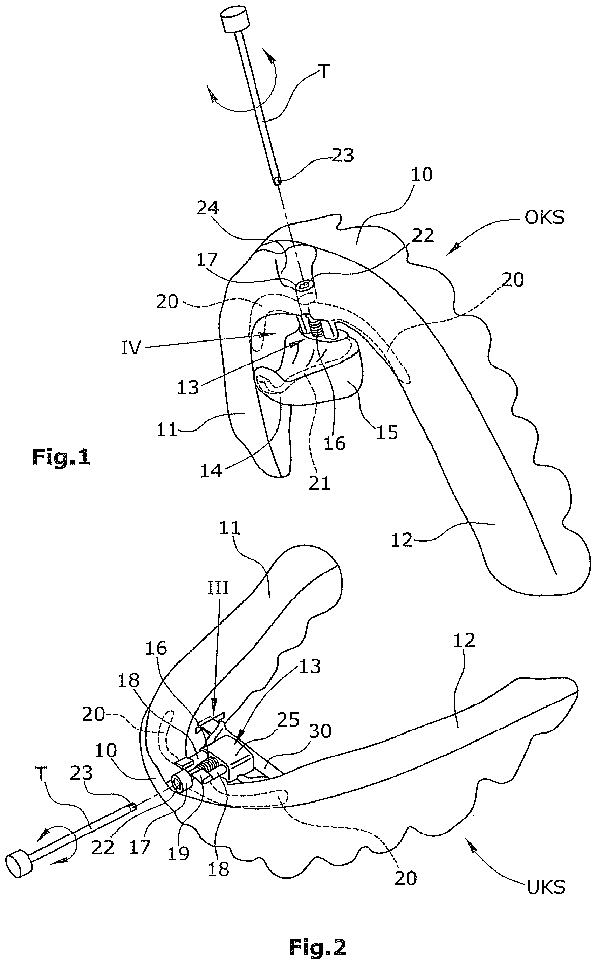

Wie Figur 1 zeigt, ist der die Kopplungsstruktur 17 bildende Spindelkopf, der einen Innensechskant 22 enthält, durch frontales Ansetzen eines Werkzeugs T mit Außensechskant 23 zugänglich. Das Werkzeug T wird in Längsrichtung der Spindel 16 angesetzt und drehfest mit dieser verbunden, um durch Drehen des Werkzeugs die Spindel zu drehen und dadurch das Kopfstück 15 mit dem Angreifteil 14 zu verschieben. Der Spindelkopf ist in einer Versenkung 24 im Kulminationspunkt des Bogenbereichs 10 angeordnet. Der Spindelkopf hat eine zylindrische glatte Außenfläche, so dass er in das Material der Kieferschiene passend und drehbar eingebettet ist.

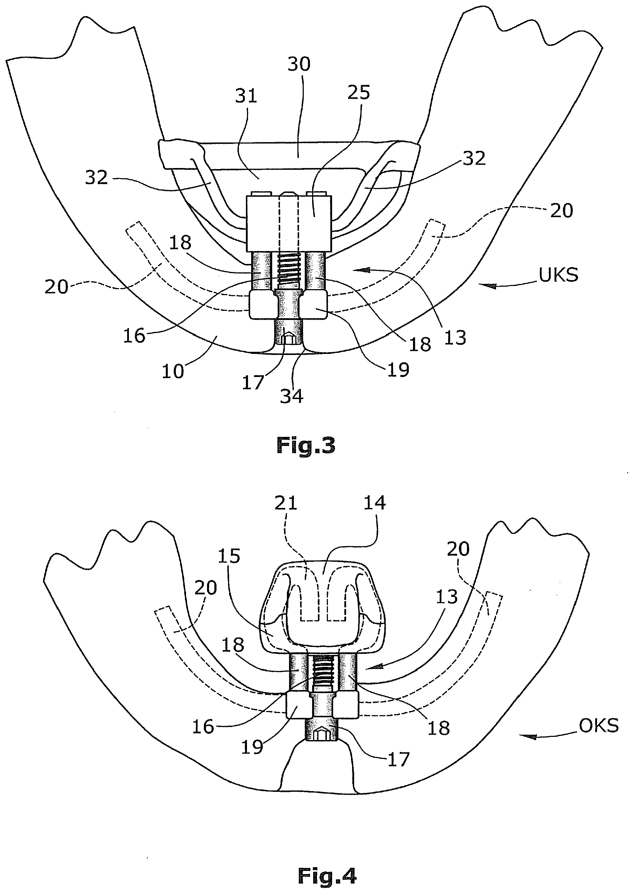

In ähnlicher Weise wie die Oberkieferschiene ist die Unterkieferschiene UKS gemäß Figuren 2 und 3 ausgebildet. Der Verstellmechanismus 13 ist hierbei der gleiche wie bei der Oberkieferschiene, wobei in den Zeichnungen (Figuren 2 und 3) auch die Spindelmutter 25 sichtbar ist, von der die beiden Führungsstifte 18 längs verschiebbar abstehen. Die Spindelmutter 25 steht dorsal von dem Bogenbereich 10 ab und ragt somit in den Bereich zwischen den Seitenschenkeln 11 und 12. Mit der Spindelmutter 25 ist ein Angreifteil 30 in Form eines Querbügels verbunden, der eine Öse 31 zum Hindurchstecken des hakenförmigen Angreifteils 14 der Oberkieferschiene OKS bildet. Der Querbügel 30 ist mit Armen 32 mit der Spindelmutter 25 verbunden. Auch bei dieser Unterkieferschiene ist das Joch 19 in das Material der Kieferschiene eingebettet, während die Spindelmutter mit dem daran befestigten Eingreifteil in Längsrichtung verschiebbar ist. Der Spindelkopf befindet sich in einer Versenkung 34 im Scheitelpunkt des frontalen Bogenbereichs 10, wo er mit dem Werkzeug T zugänglich ist.

Das Angreifteil 14 bildet zusammen mit dem Querbügel 30 einen Einrastmechanismus, der bei geschlossenem Mund die Oberkieferschiene und die Unterkieferschiene zusammenhält und dennoch seitliche (laterale) Relativbewegungen ermöglicht.

Bei dem vorliegenden Ausführungsbeispiel ist sowohl die Oberkieferschiene als auch die Unterkieferschiene mit einem Verstellmechanismus versehen, der durch frontales Ansetzen eines Drehwerkzeugs betätigt werden kann. Die zusammenwirkenden Angreifteile 14 und 30 beider Kieferschienen können jeweils einzeln verstellt werden, während sich die jeweilige Kieferschiene im Mund des Patienten befindet.

Im Rahmen der Erfindung müssen nicht notwendigerweise beide Kieferschienen in der erfindungsgemäßen Weise ausgebildet sein. Es ist auch möglich, dass eine der Kieferschienen mit einem anderen Verstellmechanismus oder keinem Verstellmechanismus ausgestattet ist.

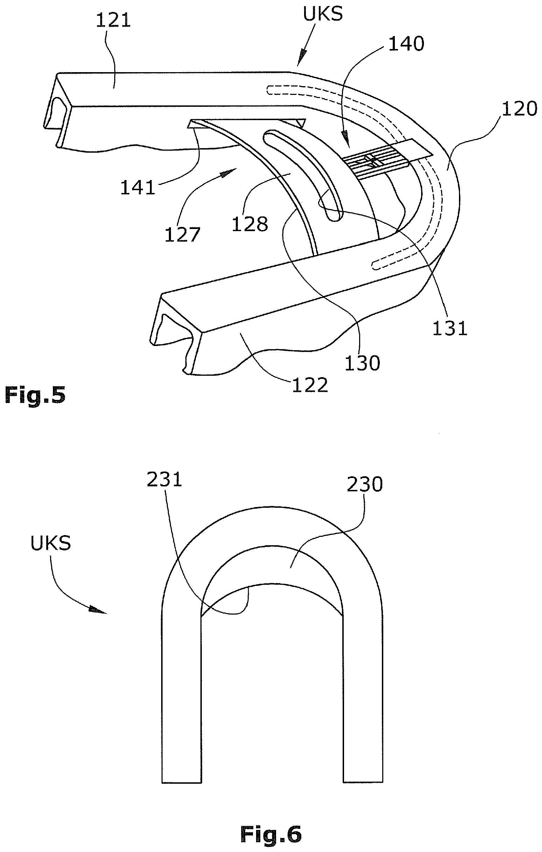

Bei dem Ausführungsbeispiel nach Fig. 5 ist eine Unterkieferhalterung 127 mit einer zweiten Schraubvorrichtung 140 an dem Bogenbereich 120 befestigt. Sie ist generell in ähnlicher Weise ausgebildet, wie bei dem ersten Ausführungsbeispiel, nämlich mit einem langgestreckten quergerichteten Ring 128 mit einer Mulde 130 an der hinteren Ringhälfte zur Führung des Steges 114 und mit einer Mulde 131 an der vorderen Ringhälfte. Die seitlichen Enden des Ringes 128 sind in Führungen 141 der Seitenschenkel 121, 122 geführt. Bei diesem Ausführungsbeispiel ist sowohl der Oberkiefersteg gemäß Fig. 1 mit dem Verstellmechanismus 13 in sagittaler Richtung verstellbar, als auch die Unterkiefer-Halterung 127 mit der Schraubvorrichtung 140.

Das Ausführungsbeispiel nach Fig. 6 unterscheidet sich von dem Ausführungsbeispiel nach Fig. 5 dadurch, dass das Angreifteil 230 nicht als langgestreckter quer gerichteter Ring 128 mit Mulden 130, 131 ausgebildet ist, sondern vielmehr als Platte in der Lateral-Sagittalebene. An dem rückwärtigen Rand 231 der Platte 230 kann das hakenförmige Angreifteil 14 angreifen. Die Platte ist fest mit der Unterkieferschiene verbunden und nicht verstellbar.

## Patentansprüche

1. Kieferorthopädischer Apparat mit einer Oberkieferschiene (OKS) und einer Unterkieferschiene (UKS), wobei mindestens eine dieser Kieferschienen ein Angreifteil (14, 30) aufweist, welches mit einem Verstellmechanismus (13) in sagittaler Richtung der Kieferschiene verstellbar ist, wobei der Verstellmechanismus (13) mit einer Stellspindel (16) versehen ist, die ein Schaftende aufweist, welches am frontalen Bogenbereich (10) der Kieferschiene freiliegt und dort eine zum frontalen Ansetzen eines Drehwerkzeugs (T) geeignete Kopplungsstruktur (17) aufweist, wobei die Kopplungsstruktur (17) in dem Material der Kieferschiene versenkt angeordnet ist, ohne über die Kontur dieser Kieferschiene hinaus vorzustehen,
dass das Angreifteil (14) Bestandteil eines aus Kunstharz geformten Kopfstücks (15) ist, dessen Position durch Betätigung des Einstellmechanismus (13) nach vorne bzw. hinten stufenlos veränderbar ist, wobei sich in dem Kopfstück (15) eine Spindelmutter befindet, die eine schraubenförmige Spindel (16) aufnimmt, welche in der Medianebene der Kieferschiene verläuft, **dadurch gekennzeichnet, dass** die Spindelmutter zwei parallel abstehende Führungsstifte (18) lagert, deren vordere Enden durch ein Joch (19) verbunden sind.

2. Kieferorthopädischer Apparat nach Anspruch 1, bei welchem jede der beiden Kieferschienen einen Verstellmechanismus (13) mit einer entsprechenden Stellspindel nach Anspruch 1 aufweist.

3. Kieferorthopädischer Apparat nach Anspruch 1 oder 2, **dadurch gekennzeichnet, dass** die Kopplungsstruktur (22) ein Innenmehrkant ist.

4. Kieferorthopädischer Apparat nach einem der Ansprüche 1 - 3, **dadurch gekennzeichnet, dass** das Angreifteil (14) der einen Kieferschiene zusammen mit dem Angreifteil (30) der anderen Kieferschiene einen Rastmechanismus bildet.

5. Kieferorthopädischer Apparat nach einem der Ansprüche 1 - 4, **dadurch gekennzeichnet, dass** der Verstellmechanismus (13) ein hakenförmiges Angreifteil (14) trägt, dessen Mittelachse in der Medianebene der Kieferschiene liegt und von einer Rückseite des Bogenbereichs (10) absteht.

6. Kieferorthopädischer Apparat nach einem der vorhergehenden Ansprüche, **dadurch gekennzeichnet, dass** das Angreifteil (30, 130) in Form eines Querbügels mit einer Spindelmutter (25) verbunden ist, wobei der Querbügel eine Öse (31) zum Hindurchstecken des Angreifteils (14) der anderen Kieferschiene bildet.

7. Kieferorthopädischer Apparat nach einem der vorhergehenden Ansprüche, **dadurch gekennzeichnet, dass** das Angreifteil (14) der einen Kieferschiene zusammen mit einem Querbügel (30, 130) der anderen Kieferschiene einen Einrastmechanismus bildet, der bei geschlossenem Mund die beiden Kieferschienen zusammenhält und seitliche Relativbewegungen ermöglicht.

8. Kieferorthopädischer Apparat nach einem der vorhergehenden Ansprüche, **dadurch gekennzeichnet, dass** die Unterkieferschiene als Angreifteil (30, 130) einen länglichen Ring (28) aufweist, der eine quer zu einer Medianebene des Unterkiefer-Angreifteils verlaufende Hauptachse aufweist.

9. Kieferorthopädischer Apparat nach einem der vorhergehenden Ansprüche, **dadurch gekennzeichnet, dass** an den Seitenschenkeln (121, 122) der Unterkieferschiene Führungen (141) für seitliche Enden des Unterkieferangreifteils (30) vorgesehen sind.

10. Kieferorthopädischer Apparat nach Anspruch 1, **dadurch gekennzeichnet, dass** das Joch (19) mit zwei aus biegsamem Draht bestehenden Armen (20) verbunden ist, welche in das Kunstharzmaterial der Kieferschiene eingebettet sind.

11. Kieferorthopädischer Apparat nach Anspruch 5, **dadurch gekennzeichnet, dass** die Führungsstifte (18) in gebogene Arme (21) übergehen, die in das Kunstharzmaterial des hakenförmigen Angreifteils (14) eingebettet sind.

## Claims

1. An orthodontic apparatus with an upper jaw brace (OKS) and a lower jaw brace (UKS), wherein at least one of these jaw braces comprises an engagement part (14, 30) adjustable in the sagittal direction of the jaw brace by means of an adjustment mechanism (13), wherein the adjustment mechanism (13) is provided with an adjustment spindle (16) having a shaft end exposed in the frontal arch region (10) of the jaw brace, where it has a coupling structure (17) adapted for the application of a turning tool (T) from the front,
wherein the coupling structure (17) is arranged sunken in the material of the jaw brace, without protruding beyond the contour of this jaw brace,
wherein the engagement part (14) is part of a head element (15) made of synthetic resin, whose position is continuously variable in the frontward and backward directions by actuation of the adjustment mechanism (13), wherein a spindle nut is situated in the head element (15), which receives a screw-shaped spindle (16) that extends in the median plane of the jaw brace,
**characterized in that**
the spindle nut supports two guide pins (18) projecting in parallel, the front ends thereof being connected by a yoke (19).

2. The orthodontic apparatus of claim 1, **characterized in that** each of the two jaw braces has an adjustment mechanism (13) with a corresponding adjustment spindle according to claim 1.

3. The orthodontic apparatus of claim 1 or 2, **characterized in that** the coupling structure (22) is a hexagon socket.

4. The orthodontic apparatus of one of claims 1 - 3, **characterized in that** the engagement part (14) of the one jaw brace forms an engagement mechanism together with the engagement part (30) of the other jaw brace.

5. The orthodontic apparatus of one of claims 1 - 4, **characterized in that** the adjustment mechanism (13) carries a hook-shaped engagement part (14) whose central axis lies in the median plane of the jaw brace and projects from a rear side of the arch region (10).

6. The orthodontic apparatus of one of the preceding claims, **characterized in that** the engagement part (30, 130) in the form of a transversal bracket is connected with a spindle nut (25), the transversal bracket forming a loop (31) for passing the engagement part (14) of the other jaw brace therethrough.

7. The orthodontic apparatus of one of the preceding claims, **characterized in that** the engagement part (14) of the one jaw brace, together with a transversal bracket (30, 130) of the other jaw brace, forms an engagement mechanism which, in the closed state of the mouth, holds together the two jaw braces and allows for lateral relative movements.

8. The orthodontic apparatus of one of the preceding claims, **characterized in that** the lower jaw brace has an elongate ring (28) as the engagement part (30, 130), which has a major surface extending transversely to a median plane of the lower jaw engagement part.

9. The orthodontic apparatus of one of the preceding claims, **characterized in that** the lateral legs (121, 122) of the lower jaw brace are provided with guides (141) for lateral ends of the lower jaw engagement part (30).

10. The orthodontic apparatus of claim 1, **characterized in that** the yoke (19) is connected with two arms (20) of flexible wire, which are embedded in the synthetic resin material of the jaw brace.

11. The orthodontic apparatus of claim 5, **characterized in that** the guide pins (18) pass into bent arms (21) embedded in the synthetic resin material of the hook-shaped engagement part (14).

## Revendications

1. Appareil orthodontique doté d'un rail de mâchoire supérieure (RMS) et d'un rail de mâchoire inférieure (RMI), dans lequel au moins un de ces rails comporte une partie de fixation (14, 30), laquelle est ajustable dans la direction sagittale du rail de mâchoire au moyen d'un mécanisme ajusteur (13), dans lequel le mécanisme ajusteur (13) est prévu avec une broche de réglage (16) comportant une tige, laquelle est exposée sur la zone courbe frontale (10) du rail de mâchoire et comporte une structure de couplage (17) adaptée pour la fixation frontale d'un outil de tournage (T), dans lequel la structure de couplage (17) est arrangée pour être coulée dans le matériau du rail de mâchoire, sans faire saillie sur le contour de ce rail de mâchoire, de sorte que la partie de fixation (14) est un composant d'une coiffe (15) formée de résine synthétique, dont la position vers l'avant ou vers l'arrière est modifiable en continu par actionnement du mécanisme ajusteur (13), dans lequel un écrou de broche se trouve dans la coiffe (15), accueillant une broche filetée (16), laquelle court dans le plan médian du rail de mâchoire, **caractérisé en ce que** l'écrou de broche porte deux broches de guidage (18) placées parallèlement, et dont les extrémités avant sont connectées par un joug (19).

2. Appareil orthodontique selon la revendication 1, dans lequel chacun des deux rails de mâchoire comporte un mécanisme ajusteur (13) doté d'une broche de réglage selon la revendication 1.

3. Appareil orthodontique selon l'une des revendications 1 ou 2, **caractérisé en ce que** la structure de couplage (22) est un polygone interne.

4. Appareil orthodontique selon l'une des revendications 1 à 3, **caractérisé en ce que** la partie de fixation (14) de l'un des rails de mâchoire forme un mécanisme à crans avec la partie de fixation (30) de l'autre rail de mâchoire.

5. Appareil orthodontique selon l'une des revendications 1 à 3, **caractérisé en ce que** le mécanisme ajusteur (13) porte une partie de fixation (14) en forme de crochet, dont l'axe médian se trouve dans le plan médian du rail de mâchoire et qui dépasse d'une face arrière de la zone courbe (10).

6. Appareil orthodontique selon l'une des revendications précédentes, **caractérisé en ce que** la partie de fixation (30, 130) sous la forme d'un étrier est relié à un écrou de broche (25), dans lequel l'étrier forme un anneau (31) passant dans la partie de fixation (14) de l'autre rail de mâchoire.

7. Appareil orthodontique selon l'une des revendications précédentes, **caractérisé en ce que** la partie de fixation (14) de l'un des rails de mâchoire forme un mécanisme à crans avec un étrier (30, 130) de la partie de fixation de l'autre rail de mâchoire, maintenant ensemble les deux rails de mâchoire lorsque la bouche est fermée et permettant les mouvements relatifs latéraux.

8. Appareil orthodontique selon l'une des revendications précédentes, **caractérisé en ce que** le rail de mâchoire inférieure comporte comme partie de fixation (30, 130) un anneau oblong (28), comportant un axe principal transversal à un plan médian de la partie de fixation du rail de mâchoire inférieure.

9. Appareil orthodontique selon l'une des revendications précédentes, **caractérisé en ce que** des guides (141) pour les extrémités latérales de la partie de fixation du rail de mâchoire inférieure (30) sont prévus sur les branches latérales (121, 122) du rail de mâchoire inférieure.

10. Appareil orthodontique selon la revendication 1, **caractérisé en ce que** le joug (19) est relié à deux bras (20) en fil flexible, qui sont incrustés dans la résine synthétique du rail de mâchoire.

11. Appareil orthodontique selon la revendication 5, **caractérisé en ce que** les broches de guidage (18) passent dans les bras cintrés (21), qui sont incrustés dans la résine synthétique de la partie de fixation (14) en forme de crochet.
